Europäisches Patentamt

European Patent Office

Office européen des brevets

Publication number: **0 283 612**

**A1**

# EUROPEAN PATENT APPLICATION

Application number: **87302905.2**

Date of filing: **02.04.87**

Int. Cl.⁴ **A61F 5/441**

Priority: **16.03.87 US 25994**

Date of publication of application:
**28.09.88 Bulletin 88/39**

Designated Contracting States:
**AT BE CH DE ES FR GB GR IT LI LU NL SE**

Applicant: **Marlen Manufacturing & Development Co.**
**5150 Richmond Road**
**Bedford, Ohio 44146(US)**

Inventor: **Fenton, Leonard**
**24761 Maidstone Lane**
**Beachwood Ohio 44122(US)**

Representative: **Seaborn, George Stephen et al**
**c/o Edward Evans & Co. Chancery House**
**53-64 Chancery Lane**
**London WC2A 1SD(GB)**

A deodorizing ostomy pouch.

A deodorizing ostomy drainage receptacle comprising a flexible pouch (10) having drainage chamber defining walls (12, 14) therein and an opening (30) in one of the walls. The opening is located in an upper portion of the pouch and is capable of receiving a stoma. An aperture (40) is also desirably located in the upper portion of the pouch and permits gas to egress therefrom. A deodorant (5) is attached to the upper portion of the pouch in the vicinity of the aperture and is contained in a separate bag (45) so that the gas must pass through the deodorant before egressing from the pouch. The deodorant is generally a solid material such as activated charcoal.

FIG. 2

## A DEODORIZING OSTOMY POUCH

### FIELD OF THE INVENTION

This invention relates to ostomy drainage receptacles and, more particularly, to ostomy appliances comprising a flexible pouch for receiving discharge from the stoma of a patient and having a deodorant attached to the pouch to substantially abate odor egressing therefrom.

### BACKGROUND OF THE INVENTION

In the art of ostomy appliances, some effort has been devoted to suppressing, abating, or eliminating the often offensive odors which accompany the use of the appliance. Such odor is generally socially offensive and thus acts as a deterent to individuals from engaging in normal social activities. Although numerous ostomy drainage receptacles are known and have a stoma receiving opening therein, they do not generally relate to suppressing, abating, etc. odors emanating therefrom. Examples of such appliances include U.S. Patent No. 2,784,718; 2,595,934; 2,684,676; 3,897,780; 4,219,023; and Reissue No. 29,453.

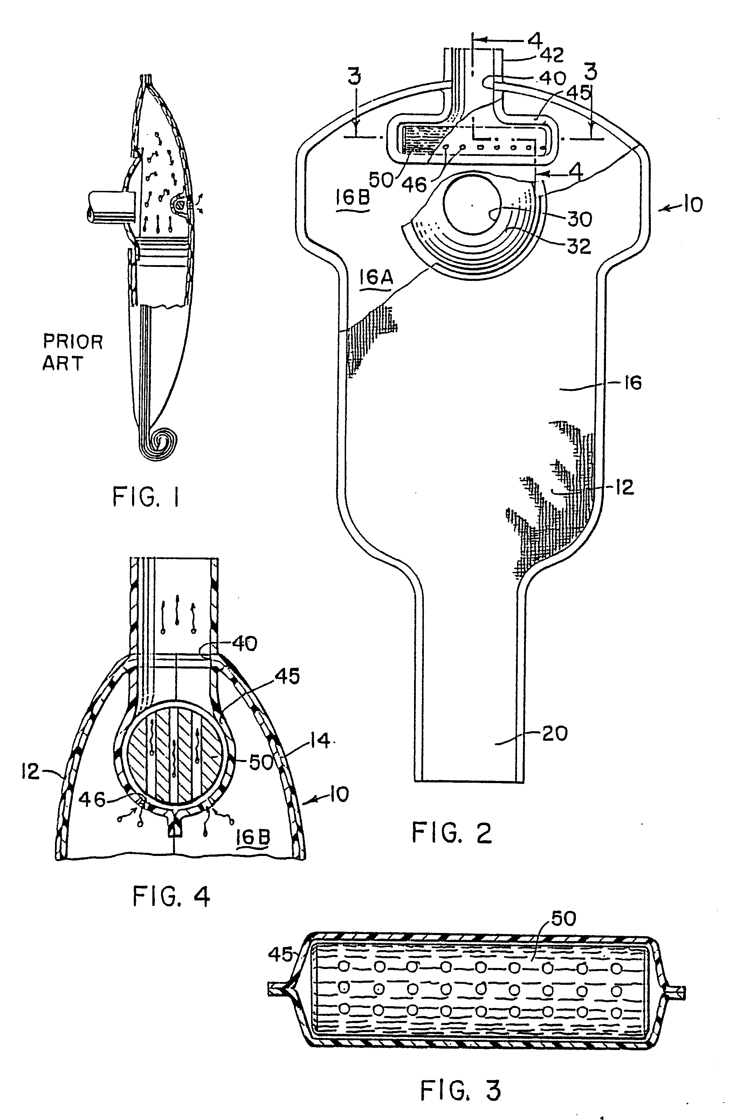

A particular prior art device is shown in Fig. 1, wherein a small pocket is sealed to the inside of the pouch walls and contains a deodorant therein such as charcoal. A small perforation permits gas to escape through the pocket. Such a device, in being located in the sidewall, has the disadvantage of permitting fluid material from the ostomy drainage pouch to escape therefrom. Moreover, a small total surface area of the deodorant is exposed to the gas within the pouch and generally does not effectively eliminate the odor.

### SUMMARY OF THE INVENTION

This invention provides a deodorant in association with an aperture located in an uppermost portion of an ostomy draining receptacle for removing offensive odors therefrom upon egress of the gas from the receptacle.

According to this invention, the receptacle comprises a flexible pouch defined by flat, flexible plastic walls sealed at their peripheries. The walls can be formed as double walls to provide a completely sealed air space between the inner and outer walls of the pouch to extend the useful life of the pouch without sacrificing comfort. An opening is provided through one of said walls or double walls adjacent to an upper portion of the pouch and a mounting assembly surrounds the opening for receiving the stoma. An aperture also exists in the upper portion of the pouch in association with a deodorant for abating odorous gas egression.

According to another aspect of this invention, the deodorant is contained in a bag which can be heat or ultrasonically sealed to the uppermost portion of the flexible pouch. The bag contains any conventional deodorant such as activated charcoal and has perforations or openings therein to permit the gas in the flexible pouch to pass through the deodorant and out of the pouch through the aperture.

According to a still further aspect of this invention, the deodorant is generally in the form of a solid such as granules or a block. An effective deodorizing ostomy flexible pouch receptacle is thus provided.

A desired aspect of this invention is that the deodorant containing bag tends to separate the upper walls of the pouch to accommodate the protruding stoma and minimize chafing of the stoma by the opposite pouch wall.

### BRIEF DESCRIPTION OF THE DRAWINGS

Fig. 1 is a cross-sectional view of the upper portion of an ostomy drainage receptacle of a prior art device.

Fig. 2 is a side-elevational view of the deodorizing ostomy receptacle of the present invention illustrating the use of a deodorant in the upper portion of the receptacle with portions of the drawing broken away for clarity.

Fig. 3 is a cross-sectional view of the deodorant contained in a bag.

Fig. 4 is a cross-sectional view taken on line 4-4 of Fig. 2.

### DETAILED DESCRIPTION OF THE INVENTION

Referring now to the drawings, an ostomy drainage receptacle generally indicated by the numeral 10 is formed by a front wall 12 and a rear wall 14 which define a drainage chamber 16. The walls 12 and 14 are generally formed from conventional materials, desirably thermoplastics such as polyethylene, polyester, nylon, and the like, and have a suitable wall thickness or alternatively, can be formed from double walls as set forth in U.S. Patent No. 3,385,298. The walls 12 and 14 are heat-sealed about their peripheries to form a leakproof seal as well as to define the chamber 16.

A drainage opening 20 exists at the bottom of the chamber and in such a location the walls 12 and 14 are not sealed. The drainage opening 20, which is desirably located at the lowest most portion of the chamber, can be sealed by a suitable clip (not shown) during usage such as set forth in U.S. Patent No. 4,460,359. Thus, the flexible pouch can be emptied whenever desired by removing the clip and permitting the contents to be drained therefrom.

The flexible pouch or drainage receptacle 10 can be attached to an individual's body in any conventional manner as known to the art and to the literature, for example as through the use of an adhesive (not shown), a belt (not shown), and the like. The pouch or drainage receptacle 10 can generally be of any size, shape, or configuration. In the particular embodiment shown, the flexible pouch has a smaller, lower chamber 16A, and a larger upper chamber 16B. As apparent from Fig. 2, the upper chamber has a wall opening 30. A mounting assembly 32 is provided in the opening to surround a stoma (not shown). The mounting assembly can be of any conventional design or structure known to the art and to the literature. An important aspect of the mounting assembly is that it is designed to conveniently receive and retain a stoma from an individual so that gas, liquid, and solid materials are collected. A problem associated with ostomy drainage receptacles or pouches is the generally unpleasant odor associated therewith. It is with regard to such odor that the aspects of the present invention are generally directed.

An aperture 40 is generally located in the upper portion of the flexible pouch and preferably in the uppermost vicinity or portion of the upper drainage chamber 16B. The aperture acts as a vent and permits gas to freely pass out of the drainage receptacle, that is the flexible pouch. The aperture, is thus, preferably located above the wall opening 30. This location ensures that the aperture is essentially free from emitting any liquid and/or solid material accumulated within the flexible pouch. The provision of the deodorant bag 45 being located in the uppermost vicinity of the drainage chamber further ensures that it will not become plugged or fouled, by intestine emitted material. An optional aspect of the present invention relates to the fact that a tube 46, for example made of a thermoplastic material, can surround the exterior portion of the aperture and extend upwardly therefrom so that it can be closed as through the utilization of a clip.

A deodorant containing bag 45 is applied to the vicinity of the aperture and internally surrounds the entire aperture so that any gas egressing from the flexible pouch must pass through the bag. The bag 45 can be applied about the aperture in any conventional manner as by heat sealing the bag there-

to, by the application of ultrasonic waves, and the like. The bag is thus generally attached to the front wall 12 as well as to the back wall 14. The bag typically has a plurality of perforations therein and can be of any size and shape to accomodate a deodorant. Naturally, the perforations are of a size to allow a gas to pass into the bag and yet retain the deodorant therein.

A deodorant 50 is generally a solid and can exist in any size or shape as for example granule, pellet, or block form. The block form generally is porous or may have holes, that is apertures, extending entirely therethrough. Various conventional materials can be utilized as the deodorant as well as materials known to the art and to the literature. A preferred deodorant is activated charcoal.

In order to deodorize the gas contained in the flexible pouch, it is desirable that the gas generally flow through the deodorant. That is, the gas must substantially or preferably pass through the deodorant before egressing through the aperture 40. Regardless of the shape of the deodorant, for example a solid block, particles, granules, or the like, the perforations 46 are thus located such that the gas, upon being admitted to the deodorizing bag 45, must generally pass through the deodorant before egressing from the bag. When a solid block is utilized, the sides of the bag 45 desirably engage the sides of the activated charcoal deodorant block 50 about the entire perimeter thereof in a gas tight engagement to prevent the gas from by-passing the deodorant. The perforations 46 are thus located in the bottom of the bag and communicate with the upper drainage chamber 16B. Gas in the flexible bag will thus pass through the bag perforations 46, through the activated charcoal where it is abated, substantially reduced, or eliminated, and then through the aperture 40 whereby it is vented to the atmosphere.

The deodorant bag can generally be of any size or shape. Desirably, the bag is elliptical such as in the shape of a pear or an egg with the deodorant being located in the wider, bottom portion thereof. An advantage of locating the bag in the upper portion of the flexible pouch is that it maintains the top of the pouch walls, that is the front wall 12 and the rear wall 14, separate from each other. Such separation is generally effective in preventing the pouch from collapsing upon itself and causing chafing or irritation to the protruding stoma. Another advantage is that the upper portion of the pouch is generally wider than the lower portion and thus provides room for a stoma to extend into the same.

The embodiment and structure of the present invention, thus, permits efficient deodorizing of substantially all of the gas egressing from the flexible pouch as well as minimizing any escape of liquid or solid matter from the draining receptacle.

Although the preferred embodiment of the invention has been shown and described, it is to be understood that various modifications and rearrangements of the parts can be resorted to without departing from the scope of the invention as disclosed and claimed herein.

## Claims

1. A deodorizing ostomy pouch comprising:

a flexible pouch having drainage chamber defining walls, a means defining an opening in one of said walls, said opening means located in an upper portion of said pouch, said opening means capable of receiving a stoma, an aperture permitting gas in said pouch to egress therefrom, said aperture located in an upper portion of said pouch, and a bag containing a deodorant therein being attached to said pouch in the vicinity of said aperture.

2. A deodorizing ostomy pouch according to claim 1, wherein said deodorant bag is attached to said pouch entirely about said aperture.

3. A deodorizing ostomy pouch according to claim 2, wherein said aperture is located in said flexible pouch at a position relatively above said opening means.

4. A deodorizing ostomy pouch according to claim 1, wherein said deodorant bag has at least one perforation therein communicating with the interior of said flexible pouch, and wherein said deodorant is located in said bag between said perforation and said aperture.

5. A deodorizing ostomy pouch according to claim 4, wherein said aperture is located in the uppermost portion of said flexible pouch, and wherein said deodorant bag prevents said flexible pouch from collapsing upon itself.

6. A deodorizing ostomy pouch according to claim 1, wherein the gas in said pouch must substantially pass through said deodorant before egressing through said aperture, and wherein said upper portion of said pouch is wider than the remaining portion of said pouch.

7. A deodorizing ostomy pouch according to claim 6, wherein said pouch has a front wall and a back wall, and wherein said deodorant bag is attached to both said front wall and said back wall.

8. A deodorizing ostomy pouch according to claim 7, wherein a tube exteriorly surrounds said aperture and extends upwardly therefrom.

9. A deodorizing ostomy pouch according to claim 1, wherein said deodorant is activated charcoal.

10. A deodorizing ostomy pouch according to claim 7, wherein said deodorant is activated charcoal.

11. An ostomy pouch containing a deodorant therein, comprising:

a flexible pouch having chamber defining walls, an aperture, said aperture allowing gas in said pouch to egress therefrom, an opening, said opening capable of receiving a stoma, a deodorant bag, said deodorant bag attached to said pouch in the vicinity of said aperture, said aperture located in said flexible pouch in a position above said opening, said deodorant bag having at least one perforation therein so that gas in said pouch can communicate with said deodorant bag whereby it is substantially deodorized before egressing from said flexible pouch.

PRIOR ART

FIG. I

FIG. 2

FIG. 4

FIG. 3

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| X | EP-A-0 064 044 (SVEN HAGBERG LÄKARPRAKTIK A.B.) * Whole document * --- | 1-7,9-11 | A 61 F 5/441 |
| X | EP-A-0 130 019 (E.R. SQUIBB & SONS INC.) * Figures 1,2 * --- | 1-7,9-11 | |
| X | EP-A-0 068 964 (LABORATORIES BIOTROL S.A.) * Figures 1-3 * --- | 1-7,9-11 | |
| X | EP-A-0 116 363 (SORBEXX GmbH) * Figure 1 * ----- | 1-7,9-11 | |

TECHNICAL FIELDS SEARCHED (Int. Cl.4)

A 61 F

The present search report has been drawn up for all claims

| Place of search | Date of completion of the search | Examiner |
|---|---|---|
| THE HAGUE | 20-06-1988 | ARGENTINI A. |